# EUROPÄISCHE PATENTSCHRIFT

(11) **EP 1 527 795 B1**
(45) Veröffentlichungstag und Bekanntmachung des Hinweises auf die Patenterteilung: **23.09.2009**
(21) Anmeldenummer: 04018989.6
(22) Anmeldetag: 11.08.2004
(51) Int. Cl.: A61M 5/24, G09F 3/03

(54) **Vorrichtung zur Identifizierung von vorgefüllten medizinischen Spritzen**
Device for identifying pre-filled medical syringes
Appareil pour l'identification de seringues medicales préremplies

(30) Priorität: 29.10.2003 DE 10350422
(43) Veröffentlichungstag der Anmeldung: 04.05.2005
(73) Patentinhaber: Arzneimittel GmbH Apotheker Vetter & Co. Ravensburg, 88212 Ravensburg (DE)
(72) Erfinder: Frasch, Eugen, 88094 Oberteuringen (DE)
(74) Vertreter: Dziewior, Joachim

(56) Entgegenhaltungen:
- WO-A-01/84542
- GB-A- 2 216 259
- US-A- 5 821 524
- US-A- 5 920 054
- US-A1- 2002 020 654

## Beschreibung

Die Erfindung betrifft eine Testvorrichtung zum Lesen und Prüfen einer Kodierung insbesondere an medizinischen Spritzen, mit einer Aufnahmehülse zum Einführen des die Kodierung tragenden Teils des Spritzenzylinders, mit einer im Innern der Aufnahmehülse angeordneten Leseeinrichtung für die Kodierung, ferner mit einer Auswerteeinheit mit einem Datenspeicher für die von der Leseeinrichtung aufgenommene Information und deren Vergleich mit der gespeicherten Sollvorgabe sowie einer Anzeigeeinheit für die Wiedergabe des Ergebnisses der erfolgten Auswertung.

In zunehmendem Maße werden Patienten dazu angeleitet, subkutan oder intramuskulär anzuwendende pharmazeutische Produkte sich selbst zu spritzen, wie dies bei an Diabetes erkrankten Patienten schon lange der Fall ist. Während bei der oralen Verabreichung von Medikamenten in Form von Tropfen oder Tabletten insbesondere auch durch die Farb- und Formgebung speziell bei Tabletten der Patient eine relativ hohe Sicherheit für gegeben ansieht, das richtige Medikament zu nehmen, ist dies bei der Verabreichung pharmazeutischer Substanzen mittels einer Spritze nicht in vergleichbarer Weise gegeben. Gerade hier hat der Patient in der Regel ein höheres Sicherheitsbedürfnis, da er meist von der schnelleren Wirksamkeit eines injiziert verabreichten Medikamentes weiß.

Aus der GB 2 216 259 ist eine Vorrichtung zum Prüfen einer Kodierung an vorgefüllten Ampullen bekannt, die eine in einer Aufnahmehülse angeordnete Leseeinrichtung sowie eine Auswerteeinheit aufweist.

Weiter beschreibt die US 5 920 054 eine medizinische Einrichtung, mit deren Hilfe die Möglichkeit geschaffen wird, die Daten über ein medizinisches Produkt vom Lieferanten zum Produktanwender, wie einem Krankenhaus oder einer anderen medizinischen Einrichtung, und wieder zurück zum Lieferanten zu erfassen und zu speichern. Dadurch soll sowohl der Verbraucher bei der Anwendung unterstützt als auch der Lieferant über den Einsatz Rückmeldungen erhalten.

Der Erfindung liegt daher die Aufgabe zugrunde, eine Testvorrichtung der eingangs genannten Art so auszugestalten, daß der Patient vor der Selbstanwendung einer Spritze insbesondere eine Überprüfung auf die Richtigkeit sowie die Originalität der darin enthaltenen pharmazeutischen Substanz durchführen kann.

Diese Aufgabe wird durch die Merkmale des Anspruchs 1 gelöst.

Der durch die Erfindung erreichte Vorteil besteht im wesentlichen darin, daß die Testvorrichtung entsprechend der durchzuführenden Prüfung durch den Einsatzadapter geeignet eingerichtet werden kann, so daß der Patient vor Applikation der Spritze auf vergleichsweise einfache Art und Weise signalisiert bekommt, ob die von ihm ausgewählte Spritze appliziert werden kann oder nicht. Die Anzeige kann hierbei auf optischem und/oder akustischem Wege erfolgen und hierzu bedarfsweise eine gerade bei älteren Patienten oftmals verminderte Seh- oder Höhrfähigkeit berücksichtigen.

Insbesondere ist es dadurch möglich, für die Überprüfung auch unterschiedlicher Spritzenformen, insbesondere Spritzengrößen, eine einheitliche Testvorrichtung verwenden zu können. Somit kann lediglich durch Austausch des Einsatzadapters eine einfache Anpassung an verschiedene Spritzen vorgenommen werden. Dies ist insbesondere auch für den Patienten von Vorteil, da er - etwa bei der Verordnung eines Medikaments in einer Spritze abweichender Größe oder sogar eines anderen Medikamentes - lediglich den Einsatzadapter auszutauschen braucht.

In vorteilhafter Weiterbildung der Erfindung kann der Einsatzadapter ein optisches Abbildungselement für die Leseeinrichtung aufweisen, um stets optimale Abbildungseigenschaften für ein sicheres Auslesen der Codierung zu gewährleisten.

Ebenso besteht die Möglichkeit, daß der Einsatzadapter eine Beleuchtungseinrichtung für die Codierung aufweist.

Ferner wird im Rahmen der Erfindung vorgeschlagen, daß der Einsatzadapter oder die Aufnahmehülse eine Druckeinrichtung zum Aufbringen einer Zusatzcodierung auf den Spritzenzylinder aufweist. Diese Druckeinrichtung kann beispielsweise in der Art eines Tintenstrahl-Druckkopfes ausgebildet sein.

Der Datenspeicher kann ein fester Bestandteil der Auswerteeinheit und/oder als externes Speichermodul ausgebildet sein. Enthält die Testvorrichtung lediglich einen festen Datenspeicher, so wird dieser in der Regel fest mit einem bestimmten Medikament korreliert sein. Ist dagegen zusätzlich oder ausschließlich ein externes Speichermodul, beispielsweise in der Art eines sogenannten Memory-Stics vorgesehen, so kann dieses Speichermodul beispielsweise vom verordnenden Arzt dem Patienten ausgehändigt oder aber der Spritzenverpackung beigefügt sein. Ein solches externes Speichermodul bietet jedoch noch weiterreichende Möglichkeiten. Zum Beispiel kann dieses Speichermodul so ausgebildet sein, daß es beispielsweise zunächst Meßdaten einer Blutzuckerbestimmung erfasst und anschließend über die Testvorrichtung Einfluß auf die Dosierung der Insulingabe vornimmt.

Die Anzeigeeinheit kann in besonders einfacher Ausgestaltung als zweifarbige, vorzugsweise Rot/Grün-Leuchtanzeige ausgebildet sein. Hierbei sind alternativ oder ergänzend auch akkustische Signale denkbar.

Die Anzeigeeinheit kann jedoch auch als alphanummerisches Anzeigeelement ausgebildet sein, wodurch der Patient weitere Informationen, etwa hinsichtlich des Verfalldatums, der vorgeschriebenen Dosierung und ähnlichem erhalten kann.

Insbeondere hinsichtlich der Berücksichtigung des Verfalldatums ist es auch denkbar, daß die Auswerteeinheit eine Empfangseinheit für einen Zeitzeichensender aufweist, der aus dem aktuellem Tagesdatum und dem in der Codierung enthaltenen Verfallsdatum wiederum dem Patienten Auskunft über die noch vorhandene oder schon abgelaufene Anwendbarkeit des Medikamentes vermittelt.

Im folgenden wird die Erfindung an einem in der Zeichnung schematisch dargestellten Ausführungsbeispiel näher erläutert; es zeigen:

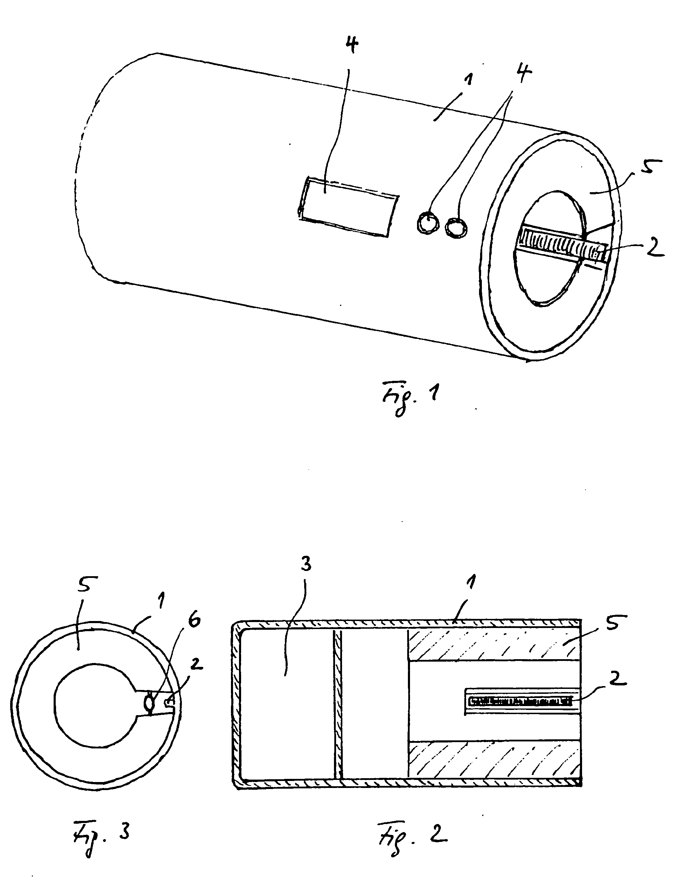

- Fig. 1: eine perspektivische Darstellung der erfindungsgemäßen Testvorrichtung,
- Fig. 2: einen Längsschnitt durch den Gegenstand nach Fig. 1.
- Fig. 3: einen Querschnitt durch den Gegenstand nach Fig. 1.

Die in der Zeichnung dargestellte Testvorrichtung dient zum Lesen und Prüfen einer Codierung insbesondere an in der Zeichnung nicht näher dargestellten medizinischen Spritzen.

Die Testvorrichtung ist mit einer Aufnahmehülse 1 versehen, die zum Einführen des die Codierung tragenden Teils des Spritzenzylinders versehen ist. Das Lesen der Codierung erfolgt durch eine im Inneren der Aufnahmehülse 1 angeordnete Leseeinrichtung 2, die als zweidimensionaler oder - in einfacherer und kostengünstigerer Ausgestaltung - als linearer optischer Abtaster ausgebildet ist.

In der Zeichnung ist ein linearer optischer Abtaster wiedergegeben, der sich in axialer Richtung der Aufnahmehülse 1 erstreckt. Bei dieser Anordnung ist es erforderlich, die Spritze nach Einführen in die Aufnahmehülse 1 um ihre Längsachse zu drehen, damit die Codierung wenigstens einmal vollständig vor dem Abtaster vorbeigeführt wird.

Auf dieses Drehen der Spritze kann verzichtet werden, wenn der lineare optische Abtaster stattdessen in radial ringförmiger Anordnung an der Innenwand der Aufnahmehülse 1 angeordnet ist, da dann der Lesevorgang bereits beim Einführen der Spritze in die Aufnahmehülse 1 erfolgen kann.

Weiter enthält die Testvorrichtung eine Auswerteeinheit 3 mit einem Datenspeicher, die die von der Leseeinrichtung 2 aufgenommene Information aufbereitet und sodann mit einer im Datenspeicher vorhandenen Sollvorgabe vergleicht.

Schließlich weist die Testvorrichtung noch eine Anzeigeeinheit 4 für die Wiedergabe des Ergebnisses der erfolgten Auswertung auf.

Um mit der Testvorrichtung auch Spritzen unterschiedlicher Größe überprüfen zu können, ist in die Aufnahmehülse ein austauschbarer, an die jeweils zu prüfende Spritze angepasster Einsatzadapter 5 vorgesehen.

Dieser Einsatzadapter 5 weist ein optisches Abbildungselement 6 für die Leseeinrichtung 2 auf, wodurch auch bei unterschiedlichem Spritzendurchmesser stets eine optimale Abbildung der Codierung auf die Leseeinrichtung 2 gewährleistet ist. Zusätzlich kann im Einsatzadapter 5 eine in der Zeichnung nicht näher dargestellte Beleuchtungseinrichtung für die Codierung vorhanden sein, die der Gestalt der Leseeinrichtung 2 angepasst ausgebildet sein sollte.

Weiter kann der Einsatzadapter 5 oder aber auch die Aufnahmehülse 1 selbst eine in der Zeichnung nicht näher dargestellte Druckeinrichtung aufweisen, die das Aufbringen einer Zusatzcodierung auf den Spritzenzylinder ermöglicht. Diese Zusatzcodierung wird ebenfalls über die Leseeinrichtung 2 erfasst und von der Auswerteeinheit 3 berücksichtigt, um kenntlich machen zu können, daß diese Spritze bereits früher einmal ausgelesen, oder sogar in anderer Weise vorbenutzt worden ist.

Der Datenspeicher kann entweder fester Bestandteil der Auswerteeinheit 3 oder aber als externes, ansteckbares Speichermodul ausgebildet sein. In letzterem Fall besteht die Möglichkeit, auf einfache Weise eine Anpassung oder Änderung der Vergleichsdaten vorzunehmen; insbesondere ermöglicht dies auch eine Anpassung an andere Darreichungsformen oder sogar andere Medikamente. Darüberhinaus kann ein externes Speichermodul auch zur Übertragung von Meßdaten, etwa einem Blutzuckermeßgerät dienen. Insbesondere ist auch eine Kombination sowohl eines festen wie auch eines externen Datenspeichers möglich.

Die Anzeige 4 kann in besonders einfacher Weise als zweifarbige, vorzugsweise Rot/Grün-Leuchtanzeige ausgebildet sein. Sofern weitergehende Informationen erwünscht sind, kann alternativ oder auch zusätzlich ein alphanummerisches Anzeigeelement vorhanden sein, das dem Patienten weitergehende Informationen vermitteln kann.

Schließlich besteht auch die in der Zeichnung ebenfalls nicht näher dargestellte Möglichkeit, die Auswerteeinheit mit einer Empfangseinheit für einen Zeitzeichensender zu versehen, wodurch die Testvorrichtung zusätzlich eine Überprüfung auf Verfallsdaten vornehmen oder sogar den Patienten an Verabreichungszeiten seines Medikamtes erinnern kann.

## Patentansprüche

1. Testvorrichtung zum Lesen und Prüfen einer Kodierung an medizinischen Spritzen, mit einer Aufnahmehülse (1) zum Einführen des die Kodierung tragenden Teils des Spritzenzylinders, mit einer im Innern der Aufnahmehülse (1) angeordneten Leseeinrichtung (2) für die Kodierung, ferner mit einer Auswerteeinheit (3) mit einem Datenspeicher für die von der Leseeinrichtung (2) aufgenommene Information und deren Vergleich mit der gespeicherten Sollvorgabe sowie einer Anzeigeeinheit (4) für die Wiedergabe des Ergebnisses der erfolgten Auswertung **dadurch gekennzeichnet, daß** die Leseeinrichtung (2) als zweidimensionaler oder linearer optischer Abtaster ausgebildet ist, wobei der Abtaster sich bei linearer Ausbildung entweder in axialer Richtung der Aufnahmehülse (1) oder aber radial in ringförmiger Anordnung an der Innenwand der Aufnahmehülse (1) erstreckt, und daß in die Aufnahmehülse (1) ein austauschbarer, an die jeweils zu prüfende Spritze angepaßter Einsatzadapter (5) eingesetzt ist.

2. Testvorrichtung nach Anspruch 1, **dadurch gekennzeichnet, daß** der Einsatzadapter (5) ein optisches Abbildungselement (6) für die Leseeinrichtung (2) aufweist.

3. Testvorrichtung nach Anspruch 1 oder 2, **dadurch gekennzeichnet, daß** der Einsatzadapter (5) eine Beleuchtungseinrichtung für die Kodierung aufweist.

4. Testvorrichtung nach einem der Ansprüche 1 bis 3, **dadurch gekennzeichnet, daß** der Einsatzadapter (5) oder die Aufnahmehülse (1) eine Druckeinrichtung zum Aufbringen einer Zusatzkodierung auf den Spritzenzylinder aufweist.

5. Testvorrichtung nach einem der Ansprüche 1 bis 4, **dadurch gekennzeichnet, daß** der Datenspeicher fester Bestandteil der Auswerteeinheit (3) und/oder als externes Speichermodul ausgebildet ist.

6. Testvorrichtung nach einem der Ansprüche 1 bis 5, **dadurch gekennzeichnet, daß** die Anzeigeeinheit (4) als zweifarbige, vorzugsweise Rot/Grün-Leuchtanzeige ausgebildet ist.

7. Testvorrichtung nach einem der Ansprüche 1 bis 5, **dadurch gekennzeichnet, daß** die Anzeigeeinheit (4) als alphanumerisches Anzeigeelement ausgebildet ist.

8. Testvorrichtung nach einem der Ansprüche 1 bis 7, **dadurch gekennzeichnet, daß** die Auswerteeinheit (4) eine Empfangseinheit für einen Zeitzeichensender aufweist.

## Claims

1. A test device for reading and verifying a coding on medical syringes, comprising a receiving sleeve (1) for introduction of the part of the syringe cylinder, that carries the coding, a reading device (2) arranged in the interior of the receiving sleeve (1) for the coding, an evaluation unit (3) having a data storage means for the information read by the reading device (2) and comparison thereof with the stored reference default and an indicator unit (4) for reproducing the result of the evaluation effected, **characterised in that** the reading device (2) is in the form of a two-dimensional or linear optical sensor, wherein the sensor in a linear configuration extends either in the axial direction of the receiving sleeve (1) or however radially in an annular arrangement at the inside wall of the receiving sleeve (1), and that a replaceable insert adaptor (5) adapted to the respective syringe to be verified is fitted into the receiving sleeve (1).

2. A test device according to claim 1 **characterised in that** the insert adaptor (5) has an optical imaging element (6) for the reading device (2).

3. A test device according to claim 1 or claim 2 **characterised in that** the insert adaptor (5) has a lighting device for the coding.

4. A test device according to one of claims 1 to 3 **characterised in that** the insert adaptor (5) or the receiving sleeve (1) has a printing device for applying an additional coding to the syringe cylinder.

5. A test device according to one of claims 1 to 4 **characterised in that** the data storage means is a fixed component of the evaluation unit (3) and/or is in the form of an external storage module.

6. A test device according to one of claims 1 to 5 **characterised in that** the indicator unit (4) is in the form of a two-coloured, preferably red/green light indicator.

7. A test device according to one of claims 1 to 5 **characterised in that** the indicator unit (4) is in the form of an alphanumeric indicator element.

8. A test device according to one of claims 1 to 7 **characterised in that** the evaluation unit (4) has a receiving unit for a time signal transmitter.

## Revendications

1. Appareil de test pour la lecture et le contrôle d'un codage de seringues médicales, comprenant un tube récepteur (1) pour l'introduction de la partie du tube de la seringue portant le codage, comprenant un dispositif de lecture (2) du codage disposé à l'intérieur du tube récepteur (1), comprenant en outre une unité de traitement (3) avec une mémoire de données pour les informations lues par le dispositif de lecture (2) et leur comparaison avec la valeur prescrite mémorisée ainsi qu'une unité d'affichage (4) pour la reproduction du résultat de l'évaluation effectuée, **caractérisé en ce que** le dispositif de lecture (2) est formé comme palpeur optique bidimensionnel ou linéaire, le palpeur s'étendant dans le cas d'une construction linéaire soit en direction axiale du tube récepteur (1) soit radialement selon une disposition annulaire à la paroi intérieure du tube récepteur (1), et **en ce qu'**un élément adaptateur (5) interchangeable correspondant à la seringue respective à contrôler est inséré dans le tube récepteur (1).

2. Appareil de test suivant la revendication 1, **caractérisé en ce que** l'élément adaptateur (5) présente un élément de reproduction optique (6) pour le dispositif de lecture (2).

3. Appareil de test suivant la revendication 1 ou 2, **caractérisé en ce que** l'élément adaptateur (5) présente un dispositif d'éclairage pour le codage.

4. Appareil de test suivant l'une des revendications 1 à 3, **caractérisé en ce que** l'élément adaptateur (5) ou le tube récepteur (1) présente un dispositif d'impression pour l'application d'un codage supplémentaire sur le tube de la seringue.

5. Appareil de test suivant l'une des revendications 1 à 4, **caractérisé en ce que** la mémoire de données est une partie fixe de l'unité de traitement (3) et/ou est formée comme module de mémoire externe.

6. Appareil de test suivant l'une des revendications 1 à 5, **caractérisé en ce que** l'unité d'affichage (4) est formée comme affichage lumineux bicolore, de préférence rouge/vert.

7. Appareil de test suivant l'une des revendications 1 à 5, **caractérisé en ce que** l'unité d'affichage (4) est formée comme élément d'affichage alphanumérique.

8. Appareil de test suivant l'une des revendications 1 à 7, **caractérisé en ce que** l'unité de traitement (4) présente une unité de réception pour un transmetteur de signal de temps.
